# DEMANDE DE BREVET EUROPEEN

(11) **EP 1 844 983 A1**
(43) Date de publication de la demande: **17.10.2007**
(21) Numéro de dépôt: 06447054.5
(22) Date de dépôt: 13.04.2006
(51) Int. Cl.: B60Q 1/44, B60Q 1/50

(54) **Dispositif support d'informations pour véhicule automobile**

(71) Demandeur: Dellil, Noureddine, 7012 Jemappes (BE)
(72) Inventeur: Dellil, Noureddine, 7012 Jemappes (BE)
(74) Mandataire: Cauchie, Daniel

(57) **Abrégé**

L'invention concerne un dispositif pour le support d'informations écrites et/ou graphiques, en particulier sécuritaires, du genre boîtier comprenant une ou plusieurs parois latérales d'un seul tenant ou non, et utilisable en relation avec un véhicule automobile.

L'invention est caractérisée en ce que le dispositif comprend une paroi (6) amovible, une paroi transparente, un élément support (15, 19) des informations écrites et/ou graphiques et au moins une source intérieure d' éclairage (20) de ces informations reliée à l'alimentation des feux stop du véhicule en sorte que lesdites informations sont éclairées lors d'une pression exercée sur la pédale du frein du véhicule.

## Description

La présente invention se rapporte, d'une manière générale, à un dispositif support d'informations pour véhicule automobile.

Plus précisément, l'invention concerne un dispositif pour le support d'informations écrites et/ou graphiques, en particulier sécuritaires, du genre boîtier comprenant une ou plusieurs parois latérales d'un seul tenant ou non, et utilisable en relation avec un véhicule automobile.

De nos jours, la densité du trafic routier pose des problèmes de sécurité de plus en plus aïgus qui nécessitent la recherche de solutions à divers niveaux. L'une d'entre elles consiste à maintenir continuellement en éveil l'attention du conducteur de manière qu'il puisse faire face au mieux à certaines situations dangereuses dont les conséquences pourraient se révéler dramatiques. Par exemple, les feux orange clignotants, les gyrophares, les feux s'allumant en cascade à l'approche de travaux ou de virages difficiles sont autant de moyens susceptibles d'attirer l'attention du conducteur d'un véhicule automobile sur un danger potentiel. Une attention particulière est donnée aux véhicules qui, dans les embarras de la circulation, sont amenés à modifier fréquemment leur vitesse de déplacement notamment en la diminuant parfois rapidement et de manière drastique. Une telle situation peut être à la source de nombreux dangers pour le ou les conducteurs qui suivent. Si l'attention de ces conducteurs est relâchée pour une raison quelconque, les risques de collision, lors d'un freinage non anticipé du conducteur qui précède, s'en trouvent accrus. Les feux stop obligatoires sur véhicules automobiles, s'ils permettent de signaler un freinage, sont toutefois incapables d'ajouter une dimension supplémentaire sous forme d'un message.

La présente invention a pour but de proposer un dispositif support d'informations écrites et/ou graphiques, en particulier sécuritaires, qui permet de pallier les inconvénients de l'état de la technique en ce qu'il est apte à signaler au conducteur d'un véhicule que le véhicule qui précède réduit sa vitesse tout en lui délivrant un message susceptible par exemple de l'inviter à une prudence accrue.

Pour atteindre ce but, le dispositif du type indiqué précédemment est caractérisé en ce qu'il comprend :
- une paroi amovible,
- une paroi transparente,
- un élément support des informations écrites et/ou graphiques éventuellement amovible,
- au moins une source intérieure d'éclairage de ces informations reliée à l'alimentation des feux stop du véhicule en sorte que lesdites informations sont éclairées lors d'une pression exercée sur la pédale du frein.

Le dispositif selon l'invention se présente sous la forme d'un boîtier généralement cubique, parallélépipédique ou cylindrique comprenant différentes parois, l'une d'elles étant amovible de manière à livrer accès à l'intérieur de ce boîtier et l'une d'elles étant transparente pour assurer un passage lumineux de l'intérieur vers l'extérieur et pour permettre une vue correcte et précise de tout objet à l'intérieur de ce boîtier.

Toutefois, selon une caractéristique particulièrement préférée, la paroi amovible est également la paroi transparente.

Selon un mode de réalisation avantageux du boîtier en question, lorsque la ou les parois latérales du boîtier ne sont pas d'un seul tenant, la paroi amovible forme l'une des parois d'un couvercle ou d'un réceptacle, l'un et l'autre étant éléments constitutifs de ce boîtier et susceptibles de solidarisation mutuelle, par exemple par emboîtement et de désolidarisation.

Chacun de ces éléments comporte une paroi latérale unique, auquel cas ces réceptacle et couvercle seront de configuration cylindrique ou, au contraire, plusieurs parois latérales, auquel cas, ceux-ci seront de forme préférentiellement cubique ou parallélépipédique.

En outre, réceptacle et couvercle présentent chacun une paroi transversale formant respectivement base et sommet.

Avantageusement, la paroi amovible d'un tel boîtier est constituée par la paroi transversale de sommet du couvercle ou de base du réceptacle. La désolidarisation de ces éléments permet de libérer cette paroi amovible.

De manière à assurer un verrouillage de l'ensemble couvercle/réceptacle et selon une autre caractéristique de l'invention, la face extérieure d'un rebord de la ou des parois latérales du réceptacle est munie d'une rainure périphérique coopérant avec un bourrelet périphérique ménagé au niveau de la face intérieure d'un rebord de la ou des parois latérales du couvercle. Inversement, la rainure périphérique peut être localisée au niveau du couvercle et le bourrelet au niveau du réceptacle.

Toutefois, selon une variante, rainure et bourrelet périphériques peuvent se limiter à des portions de rainure et à des portions de bourrelet situées en regard les unes des autres lors de l'emboîtement du réceptacle dans le couvercle de manière à assurer une coopération des portions de bourrelet avec les portions de rainures.

D'une autre manière, le verrouillage en question peut être obtenu au départ d'un bourrelet périphérique, respectivement de portions d'un tel bourrelet, ménagé au niveau de la face interne d'un rebord de la ou des parois latérales du couvercle, destiné à prendre appui sous un bourrelet périphérique, respectivement sous des portions d'un tel bourrelet, localisé au niveau de la face externe d'un rebord de la ou des parois latérales du réceptacle.

De même, selon une variante supplémentaire le couvercle, lorsqu'il est cylindrique présente un filetage au niveau de la face interne d'un rebord de sa paroi latérale dont le filet est destiné à venir en prise avec le sillon complémentaire d'un taraudage ménagé au niveau de la face externe d'un rebord de la paroi latérale du réceptacle cylindrique et ce, pour permettre le vissage de ce couvercle sur ce réceptacle.

Si nécessaire, ces verrouillages peuvent être consolidés par l'intermédiaire d'un système de fermeture supplémentaire mettant en oeuvre un élément quelconque de fermeture situé au niveau du couvercle ou du réceptacle et coopérant avec un élément de fermeture complémentaire localisé au niveau respectivement du réceptacle ou du couvercle par exemple un ensemble crochet/oeillet.

La ou les parois latérales du boîtier sont formées par la réunion de la ou des parois latérales du couvercle avec la ou les parois latérales du réceptacle après emboîtement de ceux-ci.

Toutefois, selon une variante de réalisation, la ou les parois latérales du boîtier sont chacune d'un seul tenant, c'est-à-dire exempte de solution de continuité entre les parois transversales de sommet et de base. Le boîtier ainsi configuré est, par conséquent, d'un seul tenant c'est-à-dire exempt d'éléments d'emboîtement du genre couvercle et réceptacle.

La paroi amovible d'un tel boîtier peut être solidarisée aux parois non amovibles par l'intermédiaire de moyens généralement quelconques capables d'assurer une pose et un retrait aisé de cette paroi.

De manière avantageuse, ces moyens de solidarisation sont représentés notamment par deux gouttières en regard l'une de l'autre, situées à l'intérieur du boîtier et juxtaposées à deux parois non amovibles parallèles ou situées à l'extérieur du boîtier le long des bords de deux parois non amovibles parallèles, en sorte que la paroi amovible puisse coulisser librement dans ces gouttières.

Selon un mode de réalisation particulier, les gouttières juxtaposées aux parois parallèles en question à l'intérieur du boîtier peuvent être remplacées par des rainures ménagées dans ces mêmes parois et disposées comme les gouttières.

En tout état de cause, lorsque les moyens de solidarisation en question, qu'il s'agisse de gouttières ou de rainures, sont situés à l'intérieur du boîtier, ces gouttières ou rainures seront associées à une ou deux fentes pratiquées respectivement dans l'une ou dans les deux parois non amovibles et non munies des moyens de solidarisation en question. Ces parois sont disposées généralement au droit de cette paroi amovible. En outre, les fentes seront positionnées adjacentes aux gouttières ou rainures. Par conséquent, la paroi amovible après transit par ces fentes, pourra coulisser librement dans ces gouttières ou rainures.

Le boîtier selon l'invention est avantageusement conçu en un matériau adapté à résister à des chocs éventuels à bord d'un véhicule.

Il s'agit, par exemple d'acier inoxydable, d'aluminium ou, de préférence, d'une matière synthétique polymérique répondant à ce critère.

Toutefois, la paroi transparente sera généralement élaborée à partir d'un matériau approprié répondant à cette exigence supplémentaire de transparence tel que par exemple un verre de préférence un verre de sécurité ou une matière synthétique polymérique transparente telle qu'un polyacrylate, en particulier le polyméthylméthacrylate.

Selon une caractéristique supplémentaire de l'invention, lorsque la ou les parois latérales du boîtier ne sont pas d'un seul tenant, l'élément support des informations écrites et/ou graphiques comprend habituellement une tige supportant une feuille de matériau rigide avantageusement en matière synthétique polymérique. Cette tige, fixée dans la paroi opposée à la paroi transparente par exemple la paroi de base du réceptacle du boîtier et de préférence au droit de cette paroi, comporte une extrémité libre munie d'un filet apte à coopérer avec le sillon du taraudage intérieur d'un écrou. D'autre part, la feuille, destinée à supporter elle-même les informations en question, peut être transparente, translucide ou opaque.

Lorsque celle-ci est transparente ou translucide, les informations écrites et/ou graphiques se présentent sous la forme de coloriage et/ou de dessin et/ou d'écriture réalisés à même la feuille, soit directement soit par l'intermédiaire d'un système d'impression approprié par exemple par transfert selon des techniques connues.

Toutefois, lorsque la feuille est opaque, les informations en question se présentent sous la forme d'un graphisme tel qu'un pictogramme dont le contour extérieur est découpé dans cette feuille.

D'une autre manière, les informations écrites et/ou graphiques peuvent être reproduites sur un feuillet en un matériau souple, transparent ou translucide, adapté à recevoir ces informations sous la forme de dessin et/ou de coloriage et/ou d'écriture. Ceux-ci sont réalisés à même ce feuillet, soit directement soit par l'intermédiaire d'un système d'impression approprié notamment par transfert tel que précédemment.

Ce feuillet, généralement constitué d'un matériau synthétique thermoplastique tel que le chlorure de polyvinyle (PVC), est solidarisé de manière connue à la feuille de matériau rigide par exemple par collage ou soudure telle que soudure thermique.

Un tel mode de réalisation du support des informations écrites et/ou graphiques impliquant une tige filetée surmontée d'un écrou et d'une feuille support sera utilisé de préférence en relation avec un boîtier comprenant des éléments d'emboîtement tels que réceptacle et couvercle.

Selon une variante et autre caractéristique de l'invention, lorsque la ou les parois latérales du boîtier sont d'un seul tenant, l'élément support des informations écrites et/ou graphiques est représenté par une plaque support rigide, transparente ou translucide, habituellement en un matériau synthétique polymérique tel qu'un polyacrylate, de préférence le polyméthylméthacrylate.

Cette plaque peut être solidarisée aux parois non amovibles du boîtier par l'intermédiaire de moyens aptes à assurer une pose et un retrait aisé de celle-ci.

Généralement, ces moyens de solidarisation seront analogues à ceux utilisés pour la paroi amovible, à savoir deux gouttières en regard l'une de l'autre, situées à l'intérieur du boîtier et juxtaposées à deux parois non amovibles parallèles ou situées à l'extérieur du boîtier le long des bords de deux parois non amovibles parallèles, en sorte que la plaque rigide puisse coulisser librement dans ces gouttières.

Comme dans le cas de la paroi amovible, les gouttières juxtaposées aux parois en question à l'intérieur du boîtier peuvent être remplacées par des rainures ménagées dans ces mêmes parois et disposées comme les gouttières.

En outre, comme dans le cas de la paroi amovible, lorsque les moyens de solidarisation en question, correspondent à des gouttières ou à des rainures situées à l'intérieur du boîtier, ces gouttières ou rainures seront associées à une ou deux fentes pratiquées respectivement dans l'une ou dans les deux parois non amovibles et non munies des moyens de solidarisation en question, ces parois étant disposées généralement au droit de cette paroi amovible. Cette ou ces fentes seront positionnées adjacentes aux gouttières ou rainures en relation avec la plaque rigide de manière à laisser libre passage à celle-ci.

Par conséquent, cette plaque rigide et la paroi amovible peuvent être solidarisées au boîtier, en un seul tenant, moyennant deux ensembles chacun de deux gouttières ou de deux rainures, chaque ensemble étant associé à une ou deux fentes lorsque ces gouttières ou rainures sont situées à l'intérieur du boîtier.

Toutefois, selon une caractéristique particulière et avantageuse de ce mode de réalisation, les moyens de solidarisation de la paroi amovible du boîtier et de la plaque rigide, support des informations écrites et/ou graphiques, peuvent être communs en ce qu'un seul ensemble de deux gouttières ou de deux rainures peut être prévu pour assurer le coulissement à la fois de la plaque rigide et de la paroi amovible.

Tel que décrit précédemment, les rainures se situeront à l'intérieur du boîtier alors que les gouttières pourront être localisées aussi bien à l'intérieur qu'à l'extérieur de ce boîtier.

Dans une telle configuration, uniquement une ou deux fentes s'avéreront nécessaires par lesquelles transiteront la plaque rigide et la paroi amovible.

Les informations écrites et/ou graphiques sous la forme de dessin et/ou de coloriage et/ou d'écriture sont élaborées à même cette plaque, soit directement soit par l'intermédiaire d'un système d'impression approprié selon des techniques connues.

Dans ce mode de réalisation, il est également possible de faire appel à un feuillet transparent ou translucide, en un matériau souple tel que précédemment sur lequel les informations en question, se présentant sous la forme de dessin et/ou de coloriage et/ou d'écriture, sont réalisées à même ce feuillet, soit directement soit par l'intermédiaire d'un système d'impression approprié. Ce feuillet de matériau souple est alors solidarisé à la plaque rigide par exemple par collage ou soudure telle que soudure thermique.

Un mode de réalisation du support des informations écrites et/ou graphiques de ce type c'est-à-dire faisant appel à une plaque rigide et éventuellement à un feuillet souple solidarisé à cette plaque tel que décrit précédemment est généralement réservé à un boîtier configuré en un seul tenant c'est-à-dire ne comprenant pas d'éléments d'emboîtement du genre réceptacle et couvercle.

Les informations écrites et/ou graphiques correspondent généralement à des mots simples et brefs et/ou à des graphismes ou dessins, par exemple des pictogrammes ou des représentations symboliques, aptes à délivrer un message d'interprétation aisée et non ambiguë. Ces informations peuvent être de toute nature mais seront plus généralement en relation avec la sécurité routière.

A titre d'exemples, ces informations peuvent correspondre :
- au pictogramme d'une personne handicapée. Ce pictogramme indiquera la présence d'une telle personne à bord du véhicule,
- à l'appellation « BOB » signalant que le conducteur du véhicule est affecté au transport de personnes en état d'ivresse,
- à l'appellation « GSM » signalant le danger d'utilisation d'un téléphone cellulaire dans certaines circonstances à bord d'un véhicule en mouvement,
- à la silhouette d'un oeil invitant à la prudence,
- à la reproduction d'un caducée signalant la présence d'un médecin à bord du véhicule.

La source d'éclairage est habituellement localisée et fixée au niveau de la paroi opposée à la paroi transparente, cette paroi opposée étant, de préférence, une paroi inamovible.

Cette source d'éclairage peut être constituée d'une ou de plusieurs ampoules lumineuses ou d'un ou de plusieurs tubes luminescents dont la longueur équivaut sensiblement à la longueur intérieure du boîtier. Ces ampoules ou tubes luminescents sont raccordés en série mais de préférence en dérivation. La source d'éclairage en question est avantageusement reliée par des fils conducteurs d'alimentation à une source d'énergie électrique accessible à partir du véhicule et représentée par l'alimentation des feux stop de ce véhicule en sorte qu'après mise sous tension de cette source d'éclairage, les informations écrites et/ou graphiques généralement à caractère sécuritaire, sont éclairées.

Selon une autre caractéristique de l'invention, au moins une paroi non transparente du boîtier est munie sur sa face interne d'une feuille de recouvrement en un matériau capable de réfléchir la lumière produite par la source d'éclairage et ainsi d'augmenter l'intensité lumineuse à diffuser au travers de la paroi transparente.

Il peut s'agir, par exemple d'une feuille d'aluminium ou de tout autre matériau capable de réfléchir la lumière. Cette feuille de matériau réfléchissant est posée généralement sur la face interne de la paroi supportant la source d'éclairage en question, c'est-à-dire la face tournée vers l'intérieur de ce boîtier.

Accessoirement, le boîtier selon l'invention peut être pourvu d'un système permettant la diffusion d'odeurs agréables à l'intérieur du véhicule. Généralement, ce système comprend un conteneur obturable par un bouchon amovible, par exemple un bouchon à visser. Ce conteneur contient habituellement une matière gommeuse ou un gel imprégné de liquide odorant tandis que le bouchon, qui présente un ou plusieurs orifices pour la diffusion des odeurs émises par la gomme ou le gel, est muni intérieurement d'un filtre. Si nécessaire, cette gomme peut être rechargée en liquide odorant ou simplement remplacée lorsqu'il s'avère avantageux de diffuser une odeur différente.

Le boîtier selon l'invention ainsi décrit peut être fixé au niveau du pare-brise arrière d'un véhicule automobile à l'aide de moyens connus par exemple un ensemble vis/écrou ou un système auto-collant formé de bandes adhésives auto-collantes sur leurs deux faces, l'une de ces faces étant en contact avec le boîtier, l'autre étant collée sur le support choisi. D'une autre manière, le moyen de fixation en question peut équivaloir à un système de deux bandes auto-agrippantes, telles que par exemple de type VELCRO®, l'une étant fixée au boîtier, l'autre au support faisant partie du véhicule.

En tout état de cause, le boîtier sera positionné à l'arrière du véhicule en sorte que les informations écrites et/ou graphiques étant de grandeur suffisante seront suffisamment éclairées pour être perçues et interprétées par un conducteur de véhicule situé à une distance de 5 à 10 mètres.

L'invention sera mieux comprise et d'autres buts, caractéristiques et avantages de celle-ci apparaîtront plus clairement au cours de la description explicative qui va suivre faite en référence aux dessins schématiques donnés uniquement à titre d'exemples illustrant des modes de réalisation de l'invention et dans lesquels :
- la figure 1 est une vue d'une coupe en plan d'un dispositif support conforme à l'invention, dispositif dont les parois latérales ne sont pas d'un seul tenant,
- la figure 2 est une vue frontale du dispositif à la figure 1,
- les figures 3 et 4 sont des vues de coupes en plan d'autres modes de réalisation du dispositif à la figure 1,
- les figures 5 et 6 sont des vues de coupes transversales d'un dispositif support conforme à l'invention, dispositif dont les parois latérales sont d'un seul tenant,
- la figure 7 est une vue en plan du dispositif à la figure 6.

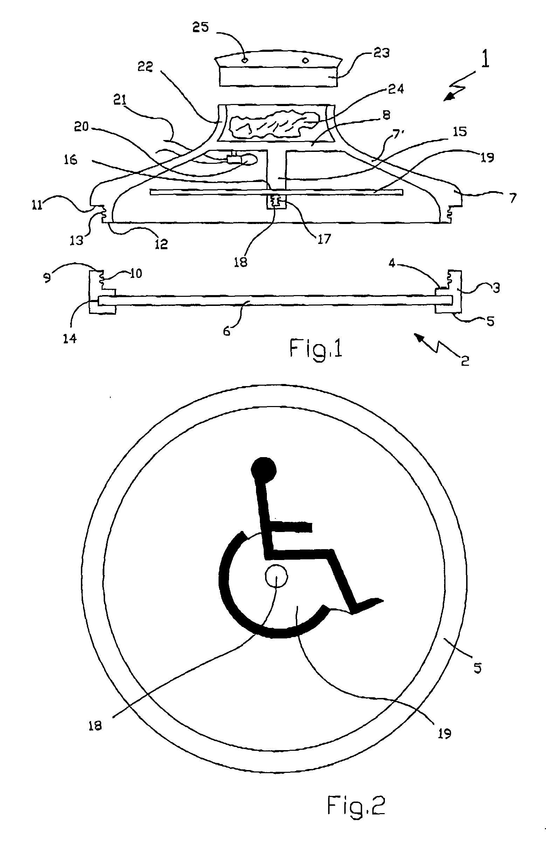

Tel que représenté à la figure 1, le dispositif selon l'invention est constitué d'un boîtier comprenant un réceptacle 1 et un couvercle 2, l'un et l'autre de forme cylindrique.

D'autre part, ce couvercle est pourvu d'une paroi 3 latérale, d'un bord 4, d'un bord 5 externe ainsi que d'une paroi 6 transversale formant sommet.

Le réceptacle, quant à lui, est muni d'une paroi 7 latérale cylindrique qui se réduit en une configuration 7' globalement tronconique se terminant par une paroi 8 transversale formant base.

Le couvercle 2 comporte, en outre, sur son bord 4, un rebord 9 externe et périphérique, un filetage 10 ayant été réalisé sur la face interne de ce rebord. D'autre part, le bord 11 du réceptacle est lui aussi pourvu d'un rebord 12 interne et périphérique dont la face externe présente un taraudage 13 complémentaire au profil du filet 10 du couvercle. On observe en outre, qu'après vissage du couvercle sur le réceptacle, les faces externes des deux parois 3/7 latérales d'une part et les faces internes de ces mêmes parois d'autre part se trouvent dans le prolongement l'une de l'autre. Le boîtier est pourvu, par conséquent d'une paroi latérale unique mais non d'un seul tenant.

Au surplus, la face interne de la paroi 3 présente une rainure 14 périphérique logeant le bord de la paroi transversale 6 représentée par une plaque transparente en PMMA.

En se référant à nouveau à la figure 1, on observe une tige 15 dont une extrémité est fixée dans la paroi transversale 8, l'autre extrémité subissant un retrait périphérique lequel détermine un bord 16. Cette extrémité présente par ailleurs, au niveau de ce retrait, un filet 17 venant en prise avec le sillon du taraudage interne d'un écrou 18 qui enserre, contre le bord 16, une feuille 19 de matière plastique rigide et opaque. Cette feuille est pré-découpée de manière à figurer une silhouette porteuse d'un message à délivrer dans le cas présent le pictogramme symbolisant un individu handicapé tel que représenté à la figure 2.

A l'arrière de cette feuille, à savoir au niveau de la paroi 8 de base, se trouve une ampoule électrique 20 de 12 V reliée par deux fils conducteurs 21 à l'un des feux stop arrières (non représentés) du véhicule.

La figure 1 montre par ailleurs, au sommet du réceptacle 1 du boîtier, un conteneur 22 obturable par un bouchon 23 et renfermant une matière gommeuse 24 imprégnée d'un liquide apte à diffuser une odeur agréable.

De manière à permettre une diffusion aisée de celle-ci, le bouchon est pourvu d'autre part de différentes orifices 25.

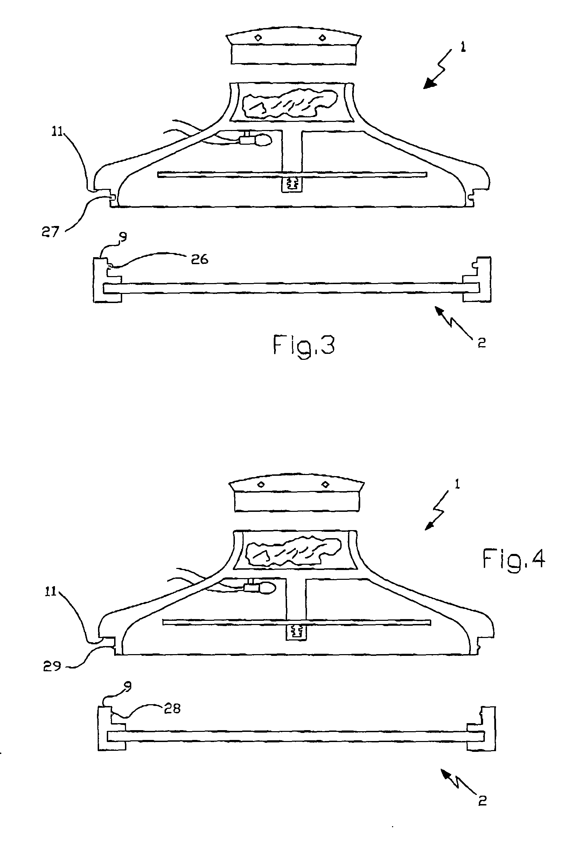

Selon un autre mode de réalisation du dispositif à la figure 1 et tel que représenté à la figure 3, le filet 10 du couvercle 2 à la figure 1 est remplacé par un bourrelet 26 périphérique alors que le réceptacle 1 est pourvu d'une rainure 27 au lieu du taraudage 13.

Une pression du rebord 9 contre le bord 11 amène le bourrelet 26 à coopérer avec la rainure 27 pour assurer la fixation du couvercle 2 sur le réceptacle 1.

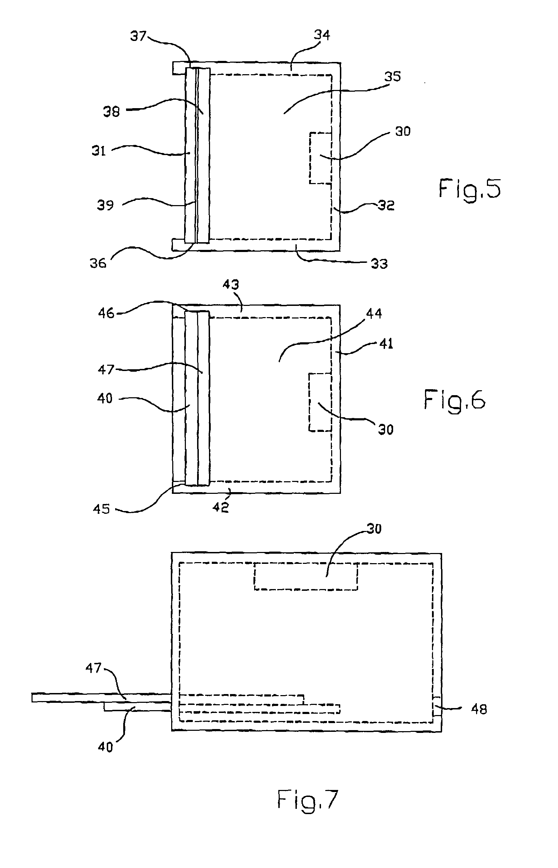

De même, dans un autre mode de réalisation à la figure 4, le filet 10 du couvercle 2 à la figure 1 et le taraudage 13 au niveau du réceptacle 1 sont remplacés chacun par un bourrelet périphérique respectivement 28 et 29. Ainsi, une pression du rebord 9 contre le bord 11 provoque l'encliquetage du bourrelet 28 sous le bourrelet 29 et, en conséquence, la fermeture du boîtier ainsi formé.

D'autre part, la figure 5 montre un dispositif selon l'invention dont le boîtier parallélépipédique renferme un système d'éclairage schématisé en 30. Ce boîtier comporte une paroi 31 amovible et transparente formant sommet, la paroi 32 opposée formant base tandis que les parois 33 et 34, latérales ainsi que la paroi 35 latérale et la paroi opposée (non visible) sont chacune d'un seul tenant. On observe, en outre, que les parois 33 et 34 se prolongent extérieurement sous la forme de gouttières respectivement 36 et 37, situées en regard l'une de l'autre. Dans ces gouttières sont logés deux bords de la paroi 31 amovible et deux bords opposés d'une plaquette 38 juxtaposée dont la face, à proximité de cette paroi 31 supporte par collage ou soudure thermique, une feuille 39 de PVC. Sur cette feuille sont reproduites, par impression, les informations écrites et/ou graphiques.

La paroi 31 et la plaquette 38 ainsi conçues peuvent coulisser librement dans les gouttières en sorte que le retrait de ces paroi et plaquette hors du boîtier assure une accessibilité à l'intérieur de celui-ci notamment au niveau du système 30 d'éclairage. De plus, la libération de la plaquette 38 du boîtier permet, si nécessaire, de modifier les informations écrites et/ou graphiques ou de les remplacer.

Ce remplacement peut s'opérer en substituant à la feuille 39 d'origine une nouvelle feuille porteuse d'autres informations écrites et/ou graphiques ou simplement en substituant à la plaquette 38 elle-même une nouvelle plaquette supportant d'autres informations de ce type.

Selon une variante de ce mode de réalisation représenté aux figures 6 et 7, le boîtier parallélépipédique comporte une paroi 40 amovible et transparente formant sommet, la paroi 41 opposée formant base tandis que les parois 42 et 43 latérales ainsi que la paroi 44 latérale et la paroi opposée (non visible) sont chacune d'un seul tenant. D'autre part, on observe deux rainures respectivement 45 et 46 ménagées dans les parois 42 et 43 et situées en regard l'une de l'autre.

Dans ces rainures sont logés deux bords opposés de la paroi 40 amovible et deux bords opposés d'une plaquette 47 juxtaposée, les informations écrites et/ou graphiques étant apposées à même cette plaquette par exemple par dessin et/ou coloriage et/ou écriture.

Par ailleurs, la paroi 40 et la plaquette 47 peuvent coulisser librement dans les rainures et s'extraire du boîtier, tel que représenté à la figure 7, par l'intermédiaire de fentes 48 situées en vis-à-vis.

Après la lecture de ce qui précède, on comprend que lors d'un freinage du véhicule en déplacement, la mise sous tension de l'ampoule électrique provoquera son allumage, l'éclairement du boîtier et la découpe sur ce fond éclairé de la silhouette obscure présentée par la feuille 19. La vue de ce pictogramme aura pour conséquence d'avertir le conducteur du véhicule qui suit par exemple à une distance de 5 à 10 mètres, de la présence d'une personne handicapée à bord du véhicule qui le précède l'invitant ainsi à redoubler de prudence.

Le dispositif selon l'invention présente plusieurs avantages susceptibles de le rendre particulièrement intéressant aussi bien lors de son utilisation que de sa réalisation. Il permet en effet d'accompagner le signal de freinage d'un véhicule automobile par une information supplémentaire essentiellement d'ordre sécuritaire. En effet, l'éclairement de cette information sous forme de message écrit et/ou graphique délivré en phase avec l'allumage des feux stop du véhicule permet d'attirer immanquablement l'attention du conducteur qui suit à relativement faible distance et ainsi de réaliser un impact visuel important, favorable à l'enregistrement de ce message par ce conducteur.

D'autre part, ce dispositif, utilisable de jour comme de nuit, est de réalisation simple et facile. Grâce à la présence d'une paroi amovible du boîtier, l'information écrite et/ou graphique peut être aisément accessible à l'intérieur de ce dispositif notamment lorsqu'il apparaît opportun de la modifier ou de la remplacer.

En outre, le couvercle et le réceptacle du boîtier peuvent être solidarisés et désolidarisés sans difficulté en raison d'un simple encliquetage à réaliser lors de l'emboîtement mutuel de ces deux éléments.

## Revendications

1. Dispositif pour le support d'informations écrites et/ou graphiques, en particulier sécuritaires, du genre boîtier comprenant une ou plusieurs parois latérales d'un seul tenant ou non, et utilisable en relation avec un véhicule automobile, **caractérisé en ce qu'**il comprend :
• une paroi (6) amovible,
• une paroi transparente,
• un élément support (15, 19) des informations écrites et/ou graphiques éventuellement amovible,
• au moins une source intérieure d'éclairage (20) de ces informations reliée à l'alimentation des feux stop du véhicule en sorte que lesdites informations sont éclairées lors d'une pression exercée sur la pédale du frein du véhicule.

2. Dispositif selon la revendication 1, **caractérisé en ce que** la paroi amovible est également la paroi (6) transparente.

3. Dispositif selon la revendication 1 ou 2, **caractérisé en ce que** lorsque la ou les parois latérales du boîtier ne sont pas d'un seul tenant, la paroi amovible constitue l'une des parois d'un couvercle (2) ou d'un réceptacle (1), l'un et l'autre susceptibles de solidarisation mutuelle, pour former le boîtier, et de désolidarisation et l'un et l'autre comprenant une ou plusieurs parois (3, 7) latérales.

4. Dispositif selon la revendication 3, **caractérisé en ce que** la face extérieure d'un rebord de la ou des parois latérales du réceptacle est munie d'une rainure (27) périphérique, respectivement de portions d'une telle rainure, coopérant avec un bourrelet (26) périphérique, respectivement avec des portions d'un tel bourrelet, ménagé au niveau de la face intérieure d'un rebord de la ou des parois latérales du couvercle, et inversement.

5. Dispositif selon la revendication 3, **caractérisé en ce que** la face interne d'un rebord de la ou des parois latérales du couvercle comporte un bourrelet (28) périphérique, respectivement des portions d'un tel bourrelet, destiné à prendre appui sous un bourrelet (29) périphérique, respectivement sous des portions d'un tel bourrelet, ménagé au niveau d'un rebord de la face externe de la ou des parois latérales du réceptacle.

6. Dispositif selon la revendication 3, **caractérisé en ce que** lorsque le couvercle et le réceptacle sont cylindriques, ce couvercle présente, au niveau de la face interne d'un rebord de sa paroi latérale, un filet apte à venir en prise avec le sillon complémentaire d'un taraudage ménagé au niveau de la face externe d'un rebord de la paroi latérale du réceptacle.

7. Dispositif selon la revendication 1. ou 2, **caractérisé en ce que** lorsque la ou les parois latérales du boîtier sont chacune d'un seul tenant, la paroi amovible et l'élément support des informations écrites et/ou graphiques peuvent être solidarisées aux parois non amovibles :
• soit
a) par un ou deux ensembles de deux gouttières, les gouttières de chaque ensemble étant en regard l'une de l'autre, situées à l'intérieur du boîtier et juxtaposées à deux parois non amovibles parallèles,
ou
b) par un ou deux ensembles de deux rainures (45, 46), les rainures de chaque ensemble étant en regard l'une de l'autre, situées à l'intérieur du boîtier et ménagées dans deux parois non amovibles parallèles,
chaque ensemble de gouttières ou de rainures étant associé à une ou deux fentes (48) pratiquées respectivement dans l'une ou dans les deux parois non amovibles et non munies des gouttières ou des rainures et disposées au droit de ladite paroi amovible,
• soit par un ou deux ensembles de deux gouttières (36, 37), les gouttières de chaque ensemble étant en regard l'une de l'autre et situées à l'extérieur du boîtier le long des bords de deux parois fixes parallèles,
en sorte que la paroi amovible et l'élément support des informations écrites et/ou graphiques puissent coulisser librement dans ces gouttières ou rainures.

8. Dispositif selon l'une des revendications 1 à 7, **caractérisé en ce que** l'élément support des informations écrites et/ou graphiques comprend:
a) lorsque les parois du boîtier ne sont pas d'un seul tenant, une tige (15) fixée dans la paroi (8) opposée à la paroi (6) transparente et dont l'extrémité libre est munie d'un filet (17) apte à coopérer avec le sillon du taraudage intérieur d'un écrou (18), cette tige supportant une feuille (19) de matériau rigide, transparent, translucide ou opaque, elle-même support des informations écrites et/ou graphiques,
b) lorsque les parois du boîtier sont d'un seul tenant, une plaque rigide, transparente ou translucide.

9. Dispositif selon la revendication 8, **caractérisé en ce que** :
a) lorsque les parois du boîtier ne sont pas d'un seul tenant :
• la feuille rigide, transparente ou translucide supporte les informations écrites et/ou graphiques se présentant sous la forme de dessin et/ou de coloriage et/ou d'écriture réalisés à même cette feuille soit directement soit par un système d'impression approprié,
• la feuille rigide et opaque supporte les informations écrites et/ou graphiques se présentant sous la forme d'un graphisme (19) dont le contour extérieur est découpé dans cette feuille,
• un feuillet en un matériau souple, transparent ou translucide supporte les informations écrites et/ou graphiques, se présentant sous la forme de dessin et/ou de coloriage et/ou d'écriture réalisés à même ce feuillet soit directement soit par un système d'impression approprié, ce feuillet étant solidarisé à la feuille rigide.
b) lorsque les parois du boîtier sont d'un seul tenant :
• la plaque rigide supporte les informations écrites et/ou graphiques se présentant sous la forme de dessin et/ou de coloriage et/ou d'écriture réalisés à même ce feuillet soit directement soit par un système d'impression approprié,
• un feuillet en un matériau souple, transparent ou translucide supporte les informations écrites et/ou graphiques se présentant sous la forme de dessin et/ou de coloriage et/ou d'écriture réalisés à même ce feuillet soit directement soit par un système d'impression approprié, ce feuillet étant solidarisé à la plaque rigide.

10. Dispositif selon une des revendications 1 à 9, **caractérisé en ce que** la source d'éclairage est fixée au niveau de la paroi opposée à la paroi transparente.

11. Dispositif selon une des revendications 1 à 10, **caractérisé en ce qu'**il est pourvu d'un système (22, 23, 24) capable de diffuser des odeurs agréables à l'intérieur du véhicule.
